# EUROPEAN PATENT SPECIFICATION

(11) **EP 0 607 252 B1**
(45) Date of publication and mention of the grant of the patent: **25.11.1998**
(21) Application number: 92921278.5
(22) Date of filing: 30.09.1992
(51) Int. Cl.: A61K 31/66, A61K 31/13, A61K 31/16, A61K 31/70, A61K 31/14, A61K 31/095, A61K 31/195, A61K 31/135, A61K 31/165, A61K 31/495, A61K 31/55

(54) **COMPOSITIONS FOR THE TREATMENT OF HIV INFECTION**
ZUSAMMENSETZUNGEN ZUR BEHANDLUNG VON HIV-INFEKTIONEN
COMPOSITIONS DESTINEES AU TRAITEMENT DES INFECTIONS DUES AU VIH

(30) Priority: 30.09.1991 US 767802; 08.04.1992 US 865219
(43) Date of publication of application: 27.07.1994
(73) Proprietor: THOENE, Jess G., Ann Arbor, MI 48103 (US)
(72) Inventor: THOENE, Jess G., Ann Arbor, MI 48103 (US)
(74) Representative: Orès, Bernard
(86) International application number: US9208156
(87) International publication number: WO9306832

(56) References cited:
- EP-A- 0 204 989
- WO-A-90/08540
- WO-A-90/14007
- WO-A-94/04185
- US-A- 3 991 190
- THE JOURNAL OF CLINICAL INVESTIGATION, vol. 93, no. 5, 1994 pages 2251-2257, A. BERGAMINI ET AL. 'CYSTAMINE POTENTLY SUPRESSES IN VITRO HIV REPLICATION IN ACUTELY AND CHRONICALLY INFECTED HUMAN CELLS'
- CLINICAL RESEARCH, vol. 40, no. 2, May 1992 page 246A J.G. THOENE 'IN VITRO EFFECTIVENESS OF AMINOTHIOLS AND DISULFIDES AGAINST HIV-1'
- NATO ASI SER., SER. A, vol. 120, 1986 pages 329-342, P.S. SARIN ET AL. 'INHIBITION OF HTLV-III REPLICATION IN CELL CULTURE'
- PROC. NATL. ACAD. SCI., vol. 88, February 1991 pages 986-990, T. KALEBIC ET AL. 'SUPPRESSION OF HUMAN IMMUNODEFICIENCY VIRUS EXPRESSION IN CHRONICALLY INFECTED MONOCYTIC CELLS BY GLUTATHIONE, GLUTATHIONE ESTER AND N-ACETYLCYSTEINE'
- CARCINOGENESIS, vol. 12, no. 2, February 1991 pages 241-247, R. DJURHUUS ET AL. 'MODULATION OF GLUTATHIONE CONTENT AND THE EFFECT ON METHIONINE AUXOTROPHY AND CELLULAR DISTRIBUTION OF HOMOCYSTEINE AND CYSTEINE IN MOUSE CELL LINES'
- ARZNEIMITTEL FORSCHUNG, vol. 36, no. II, 1986 pages 1531-1534, R.S. SCHLOF ET AL. 'TREATMENT OF HTLV-III/LAV-INFECTED PATIENTS WITH D-PENICILLAMINE'
- AIDS RESEARCH AND HUMAN RETROVIRUSES, vol. 6, no. 7, 1990 pages 919-927, S. KUBOTA ET AL. '2,3 DIMERCAPTO-1-PROPANOL INHIBITS HIV-1 tat ACTIVITY, VIRAL PRODUCTION, AND INFECTIVITY IN VITRO'
- The Merck Index, 10ed, 1985, No. 2771,2773.
- CHEMICAL ABSTRACTS, Volume 78, No. 14, issued 1973, May 14, Abstract 119665t (Columbus, Ohio, USA), OXFORD et al., "Inhibitor of the particle-associated RNA polymerase of influenza A and B viruses" (John Curtin Sch. Med Res., Aust Natl University., Canberra, Aust.) J. Gen. Virol. 1973, Vol. 18, No. 1, pp. 11-19.
- CHEMICAL ABSTRACTS, Volume 81, No. 17, issued 1974, October 28 (Columbus, Ohio, USA) Abstract 1014204, HOLTZ, GERHART, "Infectious DNA from coliphage T1. V. Detection of radiation-induced latent double strand breaks by cysteamine" (Inst. Straglenbiol., Kernforschngszent. Karlscrule, Ger.) Radiat. Environ. Biophys. 1974, Vol. 11, No. 2, pp. 157-64.
- CHEMICAL ABSTRACTS, Volume 110, No. 3, issued 1989, January 16, Abstract 181562 (Columbus, Ohio, USA), SCHROEDER et al., "Differential modulation of host cell and HIV gene expression by combinations of Avarol and ATZ in-vitro" (Inst. Physiol. Chem., Johannes Gutenberg-Univ., D-6500 Mainz, Fed. Rep. Ger.) Biochem Pharmacol., 1988, Vol. 37, No. 20, pp. 3947-52.
- PAPADOPULOS-ELEOPULOS et al., 1991, "Changes in thiols and glutamate as consequence of simian immunodeficiency virus infection", The Lancet, Vol. 338, pp. 1013-14.
- TURNER, V.F., "Reducing agents and AIDS-why are we waiting", The Medical Journal of Australia, 1990, Vol. 153, p. 502.

## Description

### Field of the Invention:

The present invention relates to the use of some compounds capable of undergoing a mixed disulfide exchange with a disulfide bond, a pharmaceutically acceptable salt thereof, or prodrug thereof for the preparation of a medicament for treating HIV infections, and diseases caused by such infections, such as AIDS, ARC and related expressions of human immunodeficiency virus (HIV), such as lymphadenopathy.

### Discussion of the Background:

Acquired immunodeficiency syndrome (AIDS) and AIDS related complex (ARC) result from infection with human immunodeficiency virus (HIV). The need for an effective treatment of AIDS, ARC and lymphadenopathy is great, due to the continuing increase of HIV infections and consequent opportunistic infections in the population. Current epidemiologic data show that infection with HIV leads to AIDS in over 90% of affected individuals within a ten-year period. The number of individuals already infected means that the number of AIDS cases will continue to increase for the foreseeable future.

AZT (zidovudine) has been approved for the treatment of AIDS and ARC. However, results are less than satisfactory. In particular, AZT therapy is known to cause severe side effects, such as anemia. In addition, there are strains of HIV-1 which are resistant to treatment with AZT.

Penicillamine has also been recommended for the treatment of HIV infections (Schulof et al, Arzneimittal Forschung, vol. 36 (10), pp. 1531-1534 (1986)). However, this treatment is complicated by the toxicity of penicillamine.

Thus, there remains a need for an effective treatment of HIV infection and AIDS, ARC, and lymphadenopathy.

### SUMMARY OF THE INVENTION

Accordingly, one object of the present invention is to provide novel medicaments for use in the for the treatment of HIV infections.

It is another object of the present invention to provide novel medicaments for use in the treatment of AIDS.

It is another object of the present invention to provide novel medicaments for use in the treatment of ARC.

It is another object of the present invention to provide novel medicaments for use in the treatment of lymphadenopathy.

These and other objects, which will become apparent during the following detailed description have been achieved by the inventors' discovery that HIV infections and diseases, such as AIDS, ARC and lymphadenopathy may be treated by administering an effective amount of a compound capable of undergoing a mixed disulfide exchange with a disulfide bond, a pharmaceutically acceptable salt thereof, or a prodrug thereof, to a patient in need thereof.

### DETAILED DESCRIPTION OF THE PREFERRED EMBODIMENTS

Thus, the present invention provides the use of a compound capable of undergoing a mixed disulfide exchange with a disulfide bond and selected from the group comprising cysteamine, cystamine, phosphocysteamine, WR2721, a pharmaceutically acceptable salt thereof, or a prodrugs thereof for treating HIV infections and diseases caused by such infections, namely Acquired Immunodeficiency Syndrome (AIDS), Acquired Immunodeficiency Related Complex (ARC) and/or lymphadenopathy. Other Examples of such compounds include N,N-dimethylcysteamine, pantetheine, pantethine, WR2721, co-enzyme A, mercaptoethylgluconamide, thiocholine, dithiothreitol (DDT), dithioerythritol (DTE), aminopropanethiol, aminobutanethiol, aminopentanethiol, and related compounds.

By the term "a compound capable of undergoing a mixed disulfide exchange with a disulfide bond" is meant a compound which takes part in the following reaction under physiological conditions

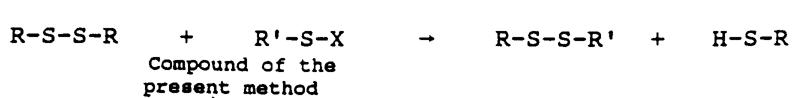

Thus, the compounds utilized in the present Invention are characterized as containing an -S-X group, in which X is -H or a group that is readily replaced by -H in the body, such as -P(=O)(OH)₂. Such compounds are collectively referred to herein after as the compounds of the present use.

Cysteamine is a known compound of the formula:

HSCH₂CH₂NH₂.

Cysteamine may be prepared from ethanolamine and carbon disulfide via 2-mercaptothiazoline as described in Gabriel et al, Ber., vol. 31, 2837 (1898); Knorr et al, Ber., vol. 36, 1281 (1903); and Mills et al, J. Am. Chem. Soc., vol. 62, 1173 (1940); or via ethyleneimine as described in Wenker, J. Am. Chem. Soc., vol. 57, 2328 (1935); Mills et al, J. Am. Chem. Soc., vol. 62, 1173 (1940); and Shirley, Preparation of Organic Intermediates, Wiley, NY, p. 189 (1951).

Cysteamine is useful for the treatment of nephropathic cystinosis: Thoene et al, The Journal of Clinical Investigation, vol. 58, pp. 180-189 (1976); Thoene et al, The Journal of Pediatrics, vol. 96, pp 1043-1044 (1980); Thoene, in Orphan Drugs and Orphan Diseases: Clinical Realities and Public Policy, Alan R. Liss, NY, pp 125-131 (1983); Thoene, in Cooperative Approaches to Research and Development of Orphan Drugs, Alan R. Liss, NY, pp. 157-162 (1985); Pisoni et al, The Journal of Biological Chemistry, vol. 260, pp. 4791-4798 (1985); Gahl et al, New England Journal of Medicine, vol. 316, pp. 971-977 (1987); and Smolin et al, Pediatric Research, vol. 23, pp. 616-620 (1988). Cysteamine is known to be safe for use in humans and does not give rise to any serious known side-effects.

Cystamine is also a known compound of the formula:

(H₂NCH₂CH₂)₂S₂.

Cystamine may be prepared by the H₂O₂ oxidation of cysteamine: Mills, Jr. et al, J. Am. Chem. Soc., vol. 62, 1173 (1940) and Barnett, J. Chem. Soc., 1944, 5.

Phosphocysteamine is the phosphorothioester of cysteamine and has the formula: 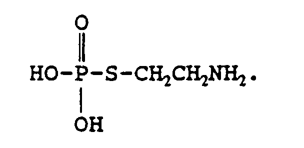 Phosphocysteamine is also known to be useful for the treatment of nephropathic cystinosis: Thoene et al, The Journal of Pediatrics, vol. 96, pp. 1043-1044 (1980); Thoene, in Cooperative Approaches to Research and Development of Orphan Drugs, Alan R. Liss, NY, pp. 157-162 (1985); and Smolin et al, Pediatric Research, vol. 23, pp. 616-620 (1988).

WR2721 has the formula:

H₂N(CH₂)₃NH(CH₂)₂SP(=O)(OH)₂

The synthesis and activity of WR2721 is described in U.S. Patent No. 3,892,824. The use of WR2721 for reducing mucin viscosity is described in Canadian Patent 1 157 774.

Thus, the medicament prepared according to the present invention may be used in a method of treating HIV infections and diseases, such as AIDS, ARC and lymphadenopathy, said method comprising or consisting of administering an effective amount of cysteamine, cystamine, phosphocysteamine, WR2721, or a pharmaceutically acceptable salt thereof to a patient in need thereof.

Although the exact dosage of cysteamine or a pharmaceutically acceptable salt thereof to be administered will vary according to the size and condition of the patient, a suitable daily dosage range for children is 1 to 3 g/m² of body surface of free base in four divided doses, preferably 1.5 to 2.5 g/m² of body surface in four divided doses, most preferably about 1.95 g/m² of body surface, in four divided doses. For adults, a suitable daily dosage may be 1 to 5g of cysteamine free base q6° (every six hours); preferably 1.5 to 2.5g, most preferably about 2g. In the case of a pharmaceutically acceptable salt, the dosage should be adjusted to result in administration of the same molar amount of cysteamine by taking into account the relative molecular weights of cysteamine and the salt thereof.

In the case of cystamine, the suitable, preferred and most preferred dosages correspond to the same respective dosages of cysteamine. In the case of the remaining thiol compounds, the suitable, preferred and most preferred dosages are selected such that the administration of the corresponding number of eqivalents of -SH delivered by administration of the above-given dosages of cysteamine is achieved, by taking into account the relative molecular weights of cysteamine and the other compound to be administered, as well as the number of thiol groups in the compound. The dosage of any disulfide in terms of mass will be the same as the corresponding thiol.

The compound of the present use, or pharmaceutically acceptable salt thereof may be suitably administered according to the present invention intravenously, parenterally, or orally. Oral administration is preferred. The compound of the present use, or pharmaceutically acceptable salt thereof may be administered in any conventional form such as a pharmaceutical composition. Suitable pharmaceutical compositions are those containing, in addition to the compound of the present method, or pharmaceutically acceptable salt thereof, a pharmaceutically acceptable carrier, such as water, starch, sugar, etc. The composition may also contain flavoring agents and may take the form of a solution, tablet, pill, capsule, etc. The ratio of the weight of compound of the present use, or pharmaceutically acceptable salt thereof to the weight of the pharmaceutical composition may, of course, vary but is suitably within 1:1 to 1:5000.

It is to be understood that the present method includes embodiments in which the compound of the present use, or pharmaceutically acceptable salt thereof is administered to a patient who is also receiving AZT, DDI or any other AIDS treatment drug. The present compound(s) and AZT or DDI may be administered to the patient in a single composition comprising both the present compounds and AZT or DDI. Alternatively, the present compound(s) and AZT or DDI may be administered separately. Further, the present method includes embodiments in which AZT or DDI is administered, without the compound of the present method, or a pharmaceutically acceptable salt thereof, for a suitable time period of hours, days, or weeks, and the AZT or DDI therapy is either preceded or followed by administration of the compound of the present use, or a pharmaceutically acceptable salt, either with or without AZT or DDI.

In another embodiment, it may be preferred to coadminister cysteine along with the compound of the present use or salt thereof, to prolong the serum half-life of the the compound of the present method or salt thereof. Of course, the present method also includes administration of mixtures of the compounds of the present use, or salts thereof.

For purposes of the present invention, the term pharmaceutically acceptable salt thereof refers to any salt of the compounds of the present use which is pharmaceutically acceptable and does not greatly reduce or inhibit the activity of the compound of the present method. Suitable examples include acid addition salts, with an organic or inorganic acid such as acetate, tartrate, trifluoroacetate, lactate, maleate, fumarate, citrate, methanesulfonate, sulfate, phosphate, nitrate, or chloride. In addition, for phosphocysteamine either or both of the hydrogen atoms on the phosphoryl group may be replaced with any suitable cation, such as Na⁺, K⁺, Mg⁺², Ca⁺⁺, NH₄⁺, or NR₄⁺ (where R is C₁₋₄ alkyl).

It is to be further understood that the compound of the present use, and pharmaceutically acceptable salts thereof include all the hydrated forms of these compounds as well as the anhydrous forms.

It is to be understood that the present method also encompasses the administration of prodrugs of the compounds of the present method. By prodrug is meant any compound that is metabolized to the compound of the present use by the body.

Thus, the compounds of the present method have now been shown to protect HIV-infected cells from the cytopathic effects of the viral infection, without exhibiting any cytotoxic effect on uninfected cells. Although not intended to be limiting in any way, a possible explanation for the efficacy of cysteamine for the treatment of HIV infections is as follows.

Human immunodeficiency virus contains coat proteins including GP120 and GP41. GP120 is a transmembrane protein which forms a domain on the exterior surface of the virus which recognizes the CD4 receptor on a subpopulation of T lymphocytes. It is thought that the recognition between the GP120 coat protein and the CD4 receptor not only leads to infection of cells by the virus but also mediates cell death by promoting autofusion, syncytia formation, and other toxic effects not yet well characterized. Crucial to the above reaction is the presence of disulfide bonds which maintain the tertiary structure of the exterior portion of GP120. It is these intrachain disulfide bonds that may be the target for cysteamine. Cysteamine is known to be highly effective in promoting intrachain disulfide scission by direct reaction with the disulfides, leading to mixed disulfide formation. Such a reaction may lead to disruption of the tertiary structure of the GP120 molecule, altering its configuration, and inhibit binding to the CD4 receptor, inhibiting viral entry, autofusion, and other toxic effects of HIV.

Although the present medicaments may be utilized to treat HIV infection at any stage, it is preferred that the treatment be initiated before the onset of frank AIDS or ARC, so that the development of frank AIDS or ARC may be prevented.

Other features of the invention will become apparent in the course of the following descriptions of exemplary embodiments which are given for illustration of the invention.

### EXAMPLES

The effectiveness of cysteamine and cystamine for the treatment of HIV infection was determined as follows.

The assay of cysteamine and cystamine was performed using the CEM human T-lymphocyte cell line as host cells, and the HTLV-III₈ strain of HIV-1 as the challenge virus. Cells were first pretreated with the test samples, then infected at a low multiplicity with virus. Twice each day an aliquot of fresh drug was added to the cultures, and the assay was monitored microscopically for signs of virus infection. Starting six days after infection, daily cell counts were performed on the cell and virus control samples to monitor the cell growth and viability. On about the seventh or eighth day postinfection, when viral CPE was maximal as determined by the cell counts, a quantitative colorimetric assay was performed to determine the extent of antiviral activity of the test samples. This assay utilized the metabolic reduction of 3-(4,5-dimethylthiazole-2-yl)-2,5-diphenyl-tetrazolium bromide (MTT) by cells surviving the virus challenge as an indication of the drug-induced suppression of viral CPE.

Cysteamine and cystamine were dissolved in a suitable solvent at a concentration of 100mM (100x for the top dose), and several aliquots were frozen at -90°C. Dilutions were made in RPMI-1640 medium containing 2mM L-glutamine and 25mM HEPES, and supplemented with 10% fetal bovine serum, 50 units of penicillin G per ml, and 50µg streptomycin sulfate per ml. Cysteamine and cystamine were assayed at concentrations of 1mM, 0.1mM, and 0.01mM.

The assay was done in 96-well tissue culture plates. A volume containing 1x10⁴ CEM cells was dispensed into each well. Each dilution of the test compound (prepared as a 4x concentration) was added to six wells of cells, and the cells were incubated at 37°C for one hour. 1000 TCID₅₀ of a frozen culture of HIV-1 was added to four of the wells for each test compound concentration. This resulted in a multiplicity of infection of 0.1 for the HIV-1 infected samples. Culture medium was added to the remaining two wells of each test compound concentration to allow evaluation of cytotoxicity. Each assay plate contained six wells of untreated, uninfected, cell control samples and six wells of untreated, infected, virus control samples. 2',3'-Dideoxyinosine (DDI) and AZT were assayed in parallel as a positive control compounds.

Assay plates were incubated at 37°C in a humidified, 5% CO₂ atmosphere. Twice each day an aliquot of a 100x cysteamine or cystamine concentrate was added to each of the assay wells. The assay plates were observed daily for signs of toxicity and for the appearance of CPE. When the CPE was maximal, samples from each assay well were processed using the colorimetric MTT assay to determine the degree of drug-induced suppression of viral CPE as well as drug cytotoxicity. Quantitation was based on the generation of MTT-formazan by the surviving cells. The results of two cytotoxicity studies and two antiviral studies are shown in tabular form below.

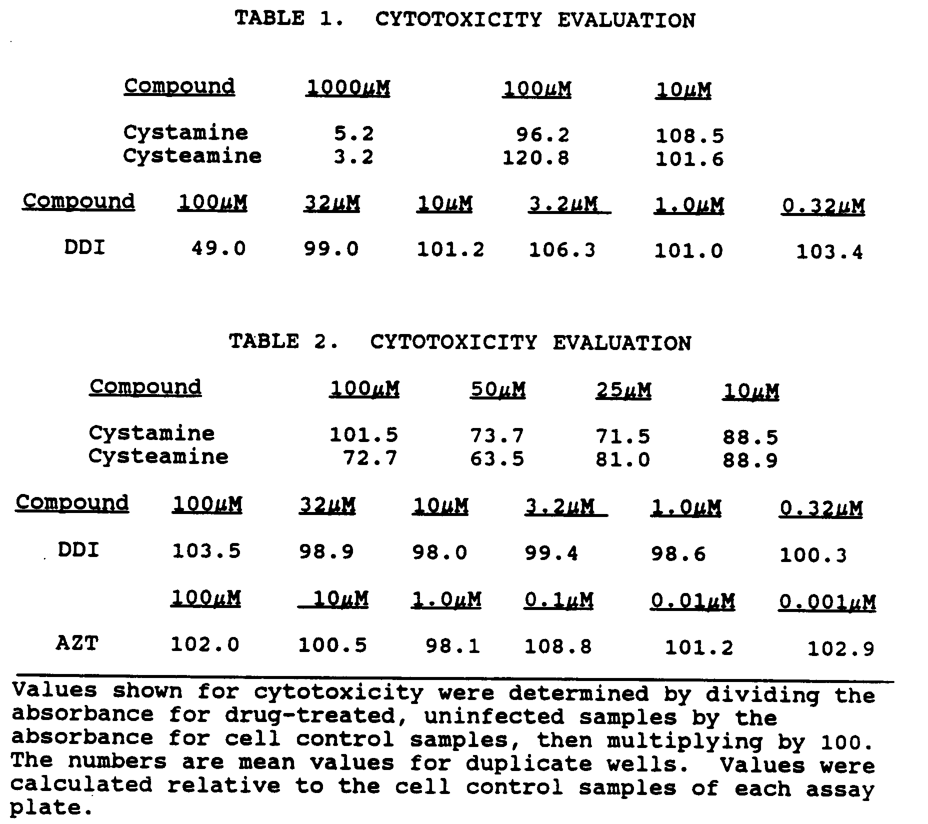

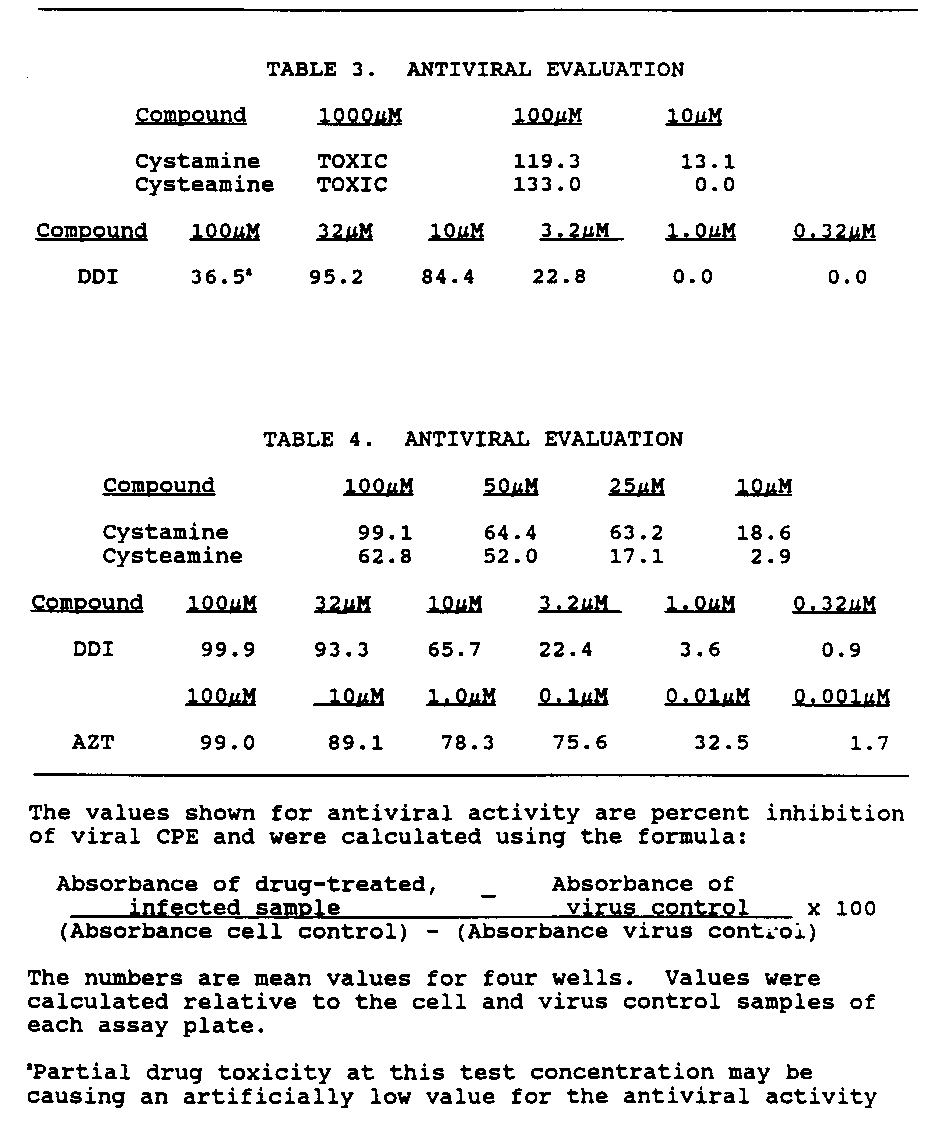

As a result of such testing it was found that although cysteamine and cystamine were toxic to the assay cells at a concentration of 1mM and exhibited little or no antiviral activity at a concentration of 0.01mM, at a concentration of 0.1mM cysteamine and cystamine were non-cytotoxic and completely protected the HIV-infected cells from the cytopathic effects of the virus infection.

## Claims

1. Use of a compound selected from the group comprising cysteamine, cystamine, phosphocysteamine, H₂N(CH₂)₃NH(CH₂)₂SP(=O)(OH)₂, pharmaceutically acceptable salts and prodrugs thereof for the preparation of a medicament for treating Human Immunodeficiency Virus (HIV) infection.

2. The use according to claim 1, wherein the medicament further comprises 3'-azido-3'-deoxythymidine (AZT) or 2',3'-dideoxyinosine (DDI).

3. The use according claim 1 or claim 2, wherein the medicament is in the form of a pharmaceutical composition comprising (i) cysteamine or cystamine or phosphocysteamine or H₂N(CH₂)₃NH(CH₂)₂SP(=O)(OH)₂ or a pharmaceutically acceptable salt or prodrug thereof and (ii) a pharmaceutically acceptable carrier.

4. The use according any one of claims 1 to 3, wherein the medicament is in the form of a pharmaceutical composition comprising (i) cysteamine or cystamine or phosphocysteamine or H₂N(CH₂)₃NH(CH₂)₂SP(=O)(OH)₂ or a pharmaceutically acceptable salt or prodrug thereof, (ii) 3'-azido-3'-deoxythymidine (AZT) or 2',3'-dideoxyinosine (DDI), and (iii) a pharmaceutically acceptable carrier.

5. The use according any one of claims 1 to 4, wherein the medicament comprises cysteamine.

6. The use according to claim 5, wherein the medicament is adapted to the administration to a child of an amount of cysteamine free base of 1 to 3 g/m² of body surface daily in four divided doses.

7. The use according to claim 6, wherein the medicament is adapted to the administration to a child of an amount of cysteamine free base of 1.5 to 2.5 g/m² of body surface daily in four divided doses.

8. The use according to claim 5, wherein the medicament is adapted to the administration to an adult of an amount of cysteamine free base of 1 to 5 g every six hours.

9. The use according to claim 8, wherein the medicament is adapted to the administration to an adult of an amount of cysteamine free base of 1.5 to 2.5 g every six hours.

10. Use of a compound selected from the group comprising cysteamine, cystamine, phosphocysteamine, H₂N(CH₂)₃NH(CH₂)₂SP(=O)(OH)₂, pharmaceutically acceptable salts or prodrugs thereof for the preparation of a medicament for treating Acquired Immunodeficiency Syndrome (AIDS), Acquired Immunodeficiency Syndrome Related Complex (ARC) and/or lymphadenopathy.

11. The use according to claim 10, wherein the medicament further comprises 3'-azido-3'-deoxythymidine (AZT) or 2',3'-dideoxyinosine (DDI).

12. The use according claim 10 or claim 11, wherein the medicament is in the form of a pharmaceutical composition comprising (i) cysteamine or cystamine or phosphocysteamine or H₂N(CH₂)₃NH(CH₂)₂SP(=O)(OH)₂ or a pharmaceutically acceptable salt or prodrug thereof, and (ii) a pharmaceutically acceptable carrier.

13. The use according any one of claims 10 to 12, wherein the medicament is in the form of a pharmaceutical composition comprising (i) cysteamine, cystamine or phosphocysteamine or H₂N(CH₂)₃NH(CH₂)₂SP(=O)(OH)₂ or a pharmaceutically acceptable salt or prodrug thereof, (ii) 3'-azido-3'-deoxythymidine (AZT) or 2',3'-dideoxyinosine (DDI), and (iii) a pharmaceutically acceptable carrier.

14. The use according any one of claims 10 to 13, wherein the medicament comprises cysteamine.

15. The use according to claim 14, wherein the medicament is adapted to the administration to a child of an amount of cysteamine free base of 1 to 3 g/m² of body surface daily in four divided doses.

16. The use according to claim 15, wherein the medicament is adapted to the administration to a child of an amount of cysteamine free base of 1.5 to 2.5 g/m² of body surface daily in four divided doses.

17. The use according to claim 15, wherein the medicament is adapted to the administration to an adult of an amount of cysteamine free base of 1 to 5 g every six hours.

18. The use according to claim 17, wherein the medicament is adapted to the administration to an adult of an amount of cysteamine free base of 1.5 to 2.5 g every six hours.

## Patentansprüche

1. Verwendung einer Verbindung, ausgewählt aus der Gruppe umfassend Cysteamin, Cystamin, Phosphocysteamin, H₂N(CH₂)₃NH(CH₂)₂SP(=O)(OH)₂, pharmazeutisch verträgliche Salze und Prodrugs davon zur Herstellung eines Medikaments zur Behandlung einer Humanen-Immunschwäche-Virus (HIV)-Infektion.

2. Verwendung nach Anspruch 1, dadurch **gekennzeichnet,** daß das Medikament ferner 3'-Azido-3'-desoxythymidin (AZT) oder 2',3'-Didesoxyinosin (DDI) umfaßt.

3. Verwendung nach Anspruch 1 oder Anspruch 2, dadurch **gekennzeichnet,** daß das Medikament in Form einer pharmazeutischen Zusammensetzung, umfassend (i) Cysteamin oder Cystamin oder Phosphocysteamin oder H₂N(CH₂)₃NH(CH₂)₂SP(=O)(OH)₂ oder ein pharmazeutisch verträgliches Salz oder ein Prodrug davon und (ii) einen pharmazeutisch verträglichen Träger, vorliegt.

4. Verwendung nach einem der Ansprüche 1 bis 3, dadurch **gekennzeichnet,** daß das Medikament in Form einer pharmazeutischen Zusammensetzung, umfassend (i) Cysteamin oder Cystamin oder Phosphocysteamin oder H₂N(CH₂)₃NH(CH₂)₂SP(=O)(OH)₂ oder ein pharmazeutisch verträgliches Salz oder ein Prodrug davon, (ii) 3'-Azido-3'-desoxythymidin (AZT) oder 2',3'-Didesoxyinosin (DDI) und (iii) einen pharmazeutisch verträglichen Träger, vorliegt.

5. Verwendung nach einem der Ansprüche 1 bis 4, dadurch **gekennzeichnet,** daß das Medikament Cysteamin umfaßt.

6. Verwendung nach Anspruch 5, dadurch **gekennzeichnet,** daß das Medikament auf die Verabreichung einer Menge freier Cysteaminbase von 1 bis 3 g/m² Körperoberfläche täglich in vier getrennten Dosen an ein Kind eingestellt ist.

7. Verwendung nach Anspruch 6, dadurch **gekennzeichnet,** daß das Medikament auf die Verabreichung einer Menge freier Cysteaminbase von 1,5 bis 2,5 g/m² Körperoberfläche täglich in vier getrennten Dosen an ein Kind eingestellt ist.

8. Verwendung nach Anspruch 5, dadurch **gekennzeichnet,** daß das Medikament auf die Verabreichung einer Menge freier Cysteaminbase von 1 bis 5 g alle sechs Stunden an einen Erwachsenen eingestellt ist.

9. Verwendung nach Anspruch 8, dadurch **gekennzeichnet,** daß das Medikament auf die Verabreichung einer Menge freier Cysteaminbase von 1,5 bis 2,5 g alle sechs Stunden an einen Erwachsenen eingestellt ist.

10. Verwendung einer Verbindung, ausgewählt aus der Gruppe umfassend Cysteamin, Cystamin, Phosphocysteamin, H₂N(CH₂)₃NH(CH₂)₂SP(=O)(OH)₂, pharmazeutisch verträgliche Salze oder Prodrugs davon zur Herstellung eines Medikaments zur Behandlung von erworbenem Immunschwäche-Syndrom (AIDS), von erworbenes Immunschwäche-Syndrom verwandtem Komplex (ARC) und/oder von Lymphadenopathie.

11. Verwendung nach Anspruch 10, dadurch **gekennzeichnet,** daß das Medikament ferner 3'-Azido-3'-desoxythymidin (AZT) oder 2',3'-Didesoxyinosin (DDI) umfaßt.

12. Verwendung nach Anspruch 10 oder Anspruch 11, dadurch **gekennzeichnet,** daß das Medikament in Form einer pharmazeutischen Zusammensetzung, umfassend (i) Cysteamin oder Cystamin oder Phosphocysteamin oder H₂N(CH₂)₃NH(CH₂)₂SP(=O)(OH)₂ oder ein pharmazeutisch verträgliches Salz oder ein Prodrug davon und (ii) einen pharmazeutisch verträglichen Träger, vorliegt.

13. Verwendung nach einem der Ansprüche 10 bis 12, dadurch **gekennzeichnet,** daß das Medikament in Form einer pharmazeutischen Zusammensetzung, umfassend (i) Cysteamin, Cystamin oder Phosphocysteamin oder H₂N(CH₂)₃NH(CH₂)₂SP(=O)(OH)₂ oder ein pharmazeutisch verträgliches Salz oder ein Prodrug davon, (ii) 3'-Azido-3'-desoxythymidin (AZT) oder 2',3'-Didesoxyinosin (DDI) und (iii) einen pharmazeutisch verträglichen Träger, vorliegt.

14. Verwendung nach einem der Ansprüche 10 bis 13, dadurch **gekennzeichnet,** daß das Medikament Cysteamin umfaßt.

15. Verwendung nach Anspruch 14, dadurch **gekennzeichnet,** daß das Medikament auf die Verabreichung einer Menge freier Cysteaminbase von 1 bis 3 g/m² Körperoberfläche täglich in vier getrennten Dosen an ein Kind eingestellt ist.

16. Verwendung nach Anspruch 15, dadurch **gekennzeichnet,** daß das Medikament auf die Verabreichung einer Menge freier Cysteaminbase von 1,5 bis 2,5 g/m² Körperoberfläche täglich in vier getrennten Dosen an ein Kind eingestellt ist.

17. Verwendung nach Anspruch 15, dadurch **gekennzeichnet,** daß das Medikament auf die Verabreichung einer Menge freier Cysteaminbase von 1 bis 5 g alle sechs Stunden an einen Erwachsenen eingestellt ist.

18. Verwendung nach Anspruch 17, dadurch **gekennzeichnet,** daß das Medikament auf die Verabreichung einer Menge freier Cysteaminbase von 1,5 bis 2,5 g alle sechs Stunden an einen Erwachsenen eingestellt ist.

## Revendications

1. Utilisation d'un composé choisi dans le groupe comprenant la cystéamine, la cystamine, la phosphocystéamine,le H₂N(CH₂)₃NH(CH₂)₂SP(=0)(OH)₂, les sels et les prodrogues de ceux-ci acceptables du point de vue pharmaceutique pour la préparation d'un médicament pour le traitement d'une infection par le virus de l'immunodéficience humaine (VIH).

2. Utilisation suivant la revendication 1, dans laquelle le médicament comprend de plus de la 3'-azido-3'-désoxythymidine (AZT) ou de la 2',3'-didésoxyinosine (DDI).

3. Utilisation suivant les revendications 1 ou 2, dans laquelle le médicament est sous la forme d'une composition pharmaceutique comprenant : (i) de la cystéamine ou de la cystamine ou de la phosphocystéamine ou du H₂N(CH₂)₃NH(CH₂)₂SP(=0)(OH)₂ ou un sel ou une prodrogue de ceux-ci acceptable du point de vue pharmaceutique, et (ii) un véhicule acceptable du point de vue pharmaceutique.

4. Utilisation suivant l'une quelconque des revendications 1 à 3, dans laquelle le médicament est sous la forme d'une composition pharmaceutique comprenant : (i) de la cystéamine ou de la cystamine ou de la phosphocystéamine ou du H₂N(CH₂)₃NH(CH₂)₂SP(=0)(OH)₂ ou un sel ou une prodrogue de ceux-ci acceptable du point de vue pharmaceutique, (ii) de la 3'-azido-3'-désoxythymidine (AZT) ou de la 2',3'-didésoxyinosine (DDI), et (iii) un véhicule acceptable du point de vue pharmaceutique.

5. Utilisation suivant l'une quelconque des revendications 1 à 4, dans laquelle le médicament comprend de la cystéamine.

6. Utilisation suivant la revendication 5, dans laquelle le médicament est adapté à l'administration à un enfant d'une quantité de cystéamine sous forme de base libre de 1 à 3 g/m² de surface corporelle par jour en quatre doses séparées.

7. Utilisation suivant la revendication 6, dans laquelle le médicament est adapté à l'administration à un enfant d'une quantité de cystéamine sous forme de base libre de 1,5 à 2,5 g/m² de surface corporelle par jour en quatre doses séparées.

8. Utilisation suivant la revendication 5, dans laquelle le médicament est adapté à l'administration à un adulte d'une quantité de cystéamine sous forme de base libre de 1 à 5 g toutes les six heures.

9. Utilisation suivant la revendication 8, dans laquelle le médicament est adapté à l'administration à un adulte d'une quantité de cystéamine sous forme de base libre de 1,5 à 2,5 g toutes les six heures.

10. Utilisation d'un composé choisi dans le groupe comprenant la cystéamine, la cystamine, la phosphocystéamine, le H₂N(CH₂)₃NH(CH₂)₂SP(=0)(OH)₂ les sels ou les prodrogues de ceux-ci acceptables du point de vue pharmaceutique pour la préparation d'un médicament pour le traitement du syndrome d'immunodéficience acquise (SIDA), du complexe apparenté au syndrome d'immunodéficience acquise (ARC) et/ou d'une lymphadénopathie.

11. Utilisation suivant la revendication 10, dans laquelle le médicament comprend de plus de la 3'-azido-3'-désoxythymidine (AZT) ou de la 2',3'-didésoxynosine (DDI).

12. Utilisation suivant les revendications 10 ou 11, dans laquelle le médicament est sous la forme d'une composition pharmaceutique comprenant : (i) de la cystéamine ou de la cystamine ou de la phosphocystéamine ou du H₂N(CH₂)₃NH(CH₂)₂SP(=0)(0H)₂ ou un sel ou une prodrogue de ceux-ci acceptable du point de vue pharmaceutique, et (ii) un véhicule acceptable du point de vue pharmaceutique.

13. Utilisation suivant l'une quelconque des revendications 10 à 12, dans laquelle le médicament est sous la forme d'une composition pharmaceutique comprenant : (i) de la cystéamine ou de la systamine ou de la phosphocystéamine ou du H₂N(CH₂)₃NH(CH₂)₂SP(=0)(0H)₂ ou un sel ou une prodrogue de ceux-ci acceptable du point de vue pharmaceutique, (ii) de la 3'-azodi-3'-désoxythymidine (AZT) ou de la 2'3'-didésoxynosine (DDI), et (iii) un véhicule acceptable du point de vue pharmaceutique.

14. Utilisation suivant l'une quelconque des revendications 10 à 13, dans laquelle le médicament comprend de la cystéamine.

15. Utilisation suivant la revendication 14, dans laquelle le médicament est adapté à l'administration à un enfant d'une quantité de cystéamine sous forme de base libre de 1 à 3 g/m² de surface corporelle par jour en quatre doses séparées.

16. Utilisation suivant la revendication 15, dans laquelle le médicament est adapté à l'administration à un enfant d'une quantité de cystéamine sous forme de base libre de 1,5 à 2,5 g/m2 de surface corporelle par jour en quatre doses séparées.

17. Utilisation suivant la revendication 15, dans laquelle le médicament est adapté à l'administration à un adulte d'une quantité de cystéamine sous forme de base libre de 1 à 5 g toutes les six heures.

18. Utilisation suivant la revendication 17, dans laquelle le médicament est adapté à l'administration à un adulte d'une quantité de cystéamine sous forme de base libre de 1,5 à 2,5 g toutes les six heures.
